# EUROPEAN PATENT SPECIFICATION

(11) **EP 0 805 698 B1**
(45) Date of publication and mention of the grant of the patent: **02.04.2003**
(21) Application number: 95910124.7
(22) Date of filing: 27.01.1995
(51) Int. Cl.: A61M 16/18

(54) **CONNECTOR WITH INTEGRAL VALVE**
VERBINDUNGSTEIL MIT INTEGRIERTEM VENTIL
CONNECTEUR A SOUPAPE INTEGREE

(43) Date of publication of application: 12.11.1997
(73) Proprietor: Abbott Laboratories, Abbott Park, Illinois 60064-3500 (US)
(72) Inventor: GRABENKORT, Richard, W., Barrington, IL 60010 (US)
(74) Representative: Modiano, Guido, Dr.-Ing.
(86) International application number: US9501007
(87) International publication number: WO96022804

(56) References cited:
- EP-A- 0 448 954
- WO-A-92/12753
- WO-A-94/15656
- CH-A- 249 699
- DE-A- 2 629 341
- GB-A- 575 539
- US-A- 2 850 041
- US-A- 4 589 445
- US-A- 5 427 145

## Description

### TECHNICAL FIELD

The present invention relates to a connector for connecting a container to a device into which liquid contents of the container are to be transferred. The connector is particularly well-suited for use in operating rooms to safely transfer an anesthetic from a container to a vaporizer while minimizing the likelihood of the anesthetic escaping to the atmosphere.

### BACKGROUND OF THE INVENTION AND TECHNICAL PROBLEMS POSED BY THE PRIOR ART

Inhalable anesthetics are typically volatile substances with relatively low boiling points and high vapor pressures. They can be flammable and explosive substances in both their liquid and vapor states. Further, inhalation of the vapor by health care personnel using them can cause drowsiness.

Therefore, such anesthetics must be safely handled in operating rooms in order to minimize the risk of inhalation by medical personnel as well as to minimize the risk of fire or explosion. Preferably, the anesthetic should be used in a way which will ensure that there is little or no release to the atmosphere at all stages of handling during normal surgical procedures.

Anesthetics are typically dispensed in liquid form to an apparatus, such as an anesthetic vaporizer, which mixes the anesthetic with oxygen and nitrous oxide. The mixture is supplied in gaseous form to the patient for inhalation.

Devices have been designed for the transfer of an anesthetic from a supply container to a vaporizer through a closed system that eliminates the escape of an anesthetic gas to the atmosphere. The devices are designed so that during set-up and disassembly procedures, a supply container of anesthetic is not open and exposed to the atmosphere in the operating room.

One system which has been developed for connecting an anesthetic container to a vaporizer employs a connector tube having adaptors at both ends. The tube is flexible and is kink-resistant. The vaporizer end of the tube is provided with a vaporizer adaptor that engages an anesthetic vaporizer. The end of the tube attached to the anesthetic container is provided with a closure adaptor that engages a closure on the anesthetic container.

The closure is preferably connected to the supply container prior to use in the operating room. The container closure has a frangible seal adapted to be perforated by a piercing means within the closure adaptor as the adaptor engages the closure. Following perforation of the frangible seal by the piercing means, the closure adaptor and closure remain locked together, and this permits the transport of anesthetic through the tube from the supply container to the vaporizer. The system remains closed to the atmosphere throughput the assembly or disassembly procedures.

Further, some types of vaporizers are intended for use with only a specific anesthetic or anesthetics. In such situations, care must be taken to insure that only the proper anesthetic is dispensed into the particular vaporizer. Connecting devices have been designed with keying systems to prevent the use of a vaporizer with an anesthetic for which it is not designed.

In particular, the anesthetic container closure has a specific shape, and the connector tube closure adaptor has a complementary shape for mating with the container closure. At the other end of the container tube, the adaptor has a special shape for mating with a complementary portion of the vaporizer anesthetic inlet port. Because the container for each type of anesthetic has its own special closure shape, and because the corresponding connector device fits only the type of vaporizer designed for that anesthetic, the probability of inadvertently using an anesthetic in an incompatible vaporizer, or of administering an incorrect anesthetic to a patient, is greatly reduced, if not eliminated.

Although such keyed, connector tubes function satisfactorily, there are inventory, installation, and management considerations associated with their use. In particular, such connector tubes are typically provided to the user initially unattached to the vaporizer or the anesthetic container. Thus, such connector tubes must be stored and maintained at an appropriate location for use, and such connector tubes can become misplaced.

Because such connector tubes are not inexpensive, it would be desirable to provide an improved connector device that is less likely to be misplaced. Further, it would be advantageous to provide an improved system which is less expensive and even easier to use.

The above-described flexible tube connector must be held and properly manipulated as the anesthetic container is attached. Further, once attached, the container must be properly inverted and maintained in a generally inverted position so as to permit the anesthetic liquid to flow into the vaporizer. During this process, the flexible tube connector must be maintained at an elevation which is at or above the vaporizer inlet, and the connector must be maintained in an orientation that does not occlude the internal flow path.

Further, after the anesthetic liquid has drained from the container into the vaporizer, care must be taken in removing the container from the connector. Proper procedures must be followed to minimize the likelihood that a significant amount of residual anesthetic liquid in the container or flexible tube connector will drip or leak out as the container is disconnected.

It would be desirable to provide an improved connector which can be readily placed in a receiving orientation wherein the anesthetic container can be easily attached to the connector. Preferably, in this orientation the connector should function in a self-maintained, closed condition wherein the connector occludes flow of the liquid anesthetic out of the vaporizer and wherein the connector also occludes the connected container to prevent escape of the anesthetic liquid or vapor.

Further, it would be advantageous if such an improved connector could readily accommodate repositioning to a self-maintained orientation for accommodating the emptying of the anesthetic container into the vaporizer while the container is in a generally inverted position.

Additionally, such an improved connector should desirably accommodate removal of the empty anesthetic container. To this end, the improved connector should preferably have a self-maintained draining position to accommodate the draining of any residual anesthetic liquid in the top of the container and/or connector into the bottom of the container prior to removal of the container.

A connector according to the preamble of claim 1 is known from EP-A-448 954.

The present invention provides an improved connector as defined in claim 1.

### SUMMARY OF THE INVENTION

A connector suitable for attaching to or mounting on an anesthetic vaporizer is provided for connecting the inlet of the vaporizer with a discharge port of a container of liquid anesthetic.

In a preferred form, the connector has a stationary coupling that is attachable, preferably permanently, to the vaporizer. The connector also has a movable coupling that is pivotally mounted to the stationary coupling. The container discharge port is connected to the movable coupling for emptying the liquid contents through a single dispensing passage defined therein.

The stationary coupling includes an internal liquid passage system having the configuration of a "Y" oriented to lie on its side with one diverging branch slanting upwardly and with one diverging branch slanting downwardly. The horizontal stem of the Y-shaped passage communicates with the vaporizer. The two branches of the "sideways" Y-shaped passage system each extend to the movable coupling.

The movable coupling can be pivoted on the stationary coupling so that the container is inverted and so that the dispensing passage in the movable coupling slants upwardly and is aligned with the end of the upwardly slanting branch of the sideways Y-shaped passage. The liquid can then drain from the container through the slanting dispensing passage of the movable coupling, through the aligned branch and stem of the Y-shaped passage in the stationary coupling, and then into the vaporizer.

After all of the container liquid has emptied into the vaporizer, the movable coupling can be pivoted downwardly on the stationary coupling so as to align the movable coupling passage with the downwardly slanting, other branch of the Y-shaped passage. In this position, the movable coupling and attached container slant downwardly from the stationary coupling. This permits any residual liquid in the passages and in the top of the container to drain to the bottom of the container before the container is disconnected.

Prior to disconnecting the container, the movable coupling can be pivoted further downwardly to move the dispensing passage away from the lower branch of the Y-shaped passage in the stationary coupling. The mating, pivot surface of the movable coupling then functions as a valve member and blocks the openings to the two branches of the Y-shaped passage. This prevents fluid from flowing into or out of the stationary coupling attached to the vaporizer.

In the preferred form, an analogous set of passages is provided for venting air into the container to facilitate the draining of'the liquid from the container.

The diverging passages of the invention need not define the specific Y-shaped passage system described above. Further, the diverging passage system could be provided in the movable coupling rather than in the stationary coupling.

Indeed, the invention can be more generally described with reference to first and second couplings having appropriate passage configurations. In particular, the first coupling can be characterized as being attachable to the vaporizer and defining a first through passage communicating at one end with the vaporizer inlet. The second coupling can be characterized as being attachable to the container and defining a second through passage communicating at one end with the container discharge port.

Either the first or second coupling can also be characterized as defining a third passage which communicates at one end with either the first or second passage.

A combination valve and mounting structure is cooperatively defined by the couplings to accommodate pivoting of the second coupling relative to the first coupling. The second coupling can be pivoted to a "closed" position occluding the first, second, and third passages.

The second coupling can also be pivoted to a raised, "fill" position establishing communication between the first and second passages whereby the anesthetic liquid can flow from the container into the vaporizer.

Finally, the second coupling can also be pivoted to a lowered, "drain" position in which the second coupling second passage slants downwardly. In the drain position, the third passage establishes communication between the first coupling first passage and the second coupling second passage.

Before lowering the second coupling to the drain position, the vaporizer inlet can be closed by a suitable valve on the vaporizer. Then, when the second coupling is moved to the drain position, any residual anesthetic liquid in the connector and/or the discharge port region on the container is permitted to drain under the influence of gravity back into the container prior to the container being disconnected from the connector.

Numerous other advantages and features of the present invention will become readily apparent from the following detailed description of the invention, from the claims, and from the accompanying drawings.

### BRIEF DESCRIPTION OF THE DRAWINGS

In the accompanying drawings that form part of the specification, and in which like numerals are employed to designate like parts throughout the same,
FIG. 1 is an exploded, perspective view of a connector of the present invention with portions of the structure broken away to illustrate interior detail;
FIG. 2 is a side elevational view taken generally along the plane 2-2 in FIG. 1, but FIG. 2 shows the components assembled with a portion of one end illustrated in cross section;
FIG. 3 is a cross-sectional view taken generally along the plane 3-3 in FIG. 2;
FIG. 4 is a fragmentary, partial, cross-sectional view similar to FIG. 2, but FIG. 4 shows the connector first coupling connected with an anesthetic vaporizer and shows the connector second coupling pivoted to a downwardly angled, closed position and partially engaged with a container;
FIG. 5 is a view similar to FIG. 4 and shows the connector fully engaged with the anesthetic container;
FIG. 6 is a view similar to FIG. 5, but FIG. 6 shows the second coupling moved to a raised, fill position;
FIG. 7 is a view similar to FIG. 6, but FIG. 7 shows the second coupling moved to a lowered, drain position; and
FIG. 8 is a view substantially identical to FIG. 5 and shows the second coupling in the lowered, closed position.

### DESCRIPTION OF THE PREFERRED EMBODIMENTS

The present invention provides a novel system for connecting an anesthetic container to a vaporizer. The system includes a connector which can, if desired, be permanently affixed to the vaporizer and which includes safety features so that the vaporizer can be connected only to a container of the type of anesthetic for which the vaporizer has been designed.

The connector is relatively inexpensive and easy to use. Connecting an anesthetic container to the vaporizer with this device results in the liquid anesthetic draining directly to the vaporizer in a closed system that eliminates the escape of anesthetic to the atmosphere.

Further, the connector, in its preferred form, has a plurality of self-maintained orientations. In one orientation the connector closes off the inlet to the vaporizer while a container full of anesthetic liquid is attached to the connector. In the closed position, the discharge port of the anesthetic container is occluded to prevent leakage or flow of the anesthetic liquid or gas.

The connector also has a fill position in which the container is generally inverted, and the connector permits the liquid anesthetic to drain from the container into the vaporizer.

Finally, the connector has a drain position in which the empty container is lower than the vaporizer fill port so that any residual anesthetic liquid in the connector and/or the container discharge region can flow back into the container before the container is removed from the connector. At this point, if desired, the vaporizer can be drained into the empty container.

While this invention is susceptible of embodiment in many different forms, this specification and the accompanying drawings disclose only some specific forms as examples of the invention. The invention is not intended to be limited to the embodiments so described, however. The scope of the invention is pointed out in the appended claims.

For ease of description, the system components of this invention are described in the normal operating position, and terms such as upper, lower, horizontal, etc., are used with reference to this position. It will be understood, however, that the components of this invention may be manufactured, stored, transported, and sold in an orientation other than the position described.

Figures illustrating the components of the invention show some mechanical elements that are known and that will be recognized by one skilled in the art. The detailed descriptions of such elements are not necessary to an understanding of the invention, and accordingly, are herein presented only to the degree necessary to facilitate an understanding of the novel features of the present invention.

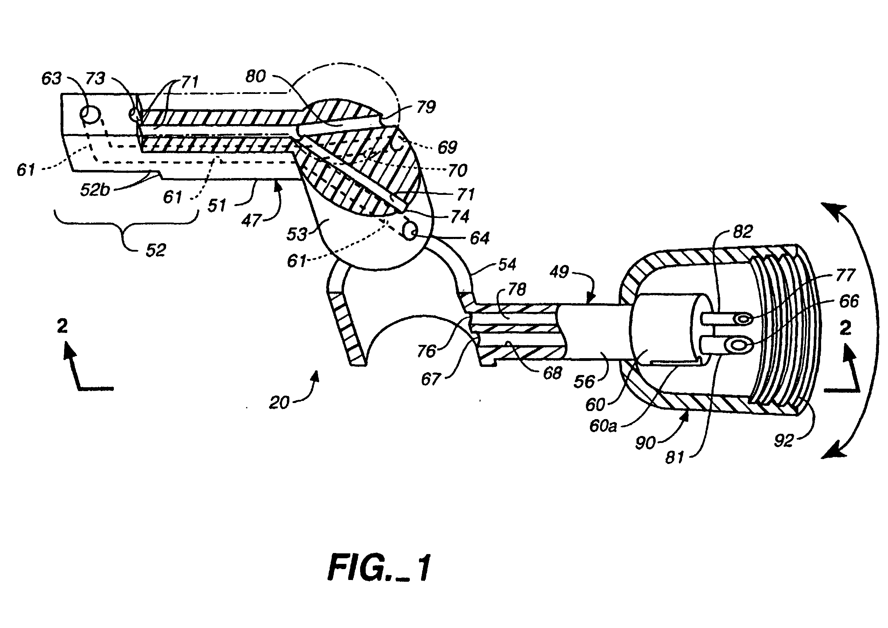

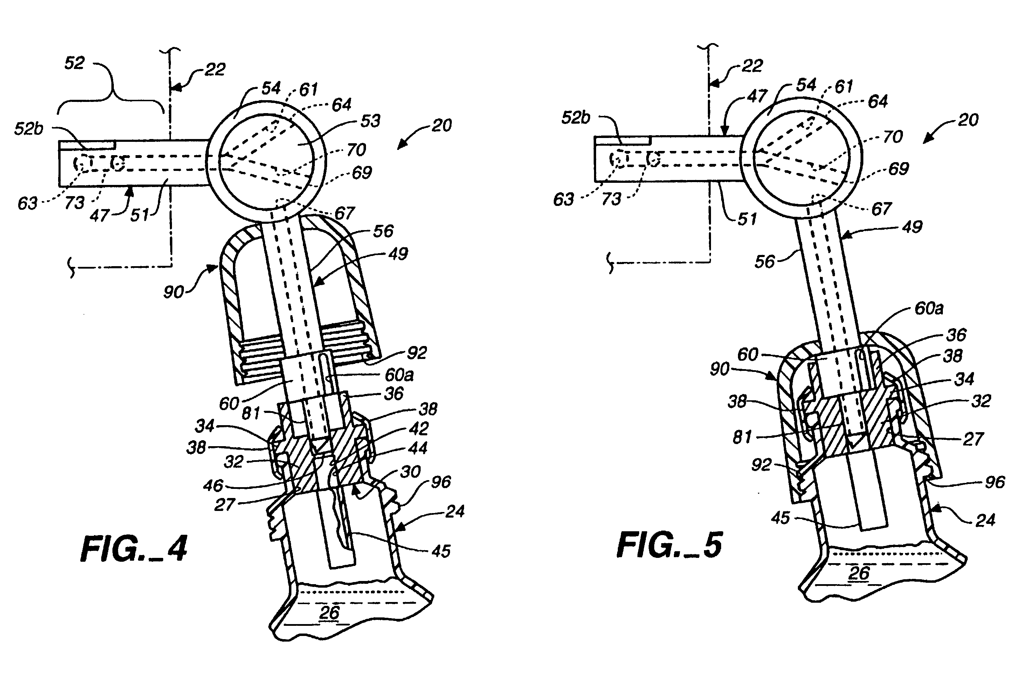

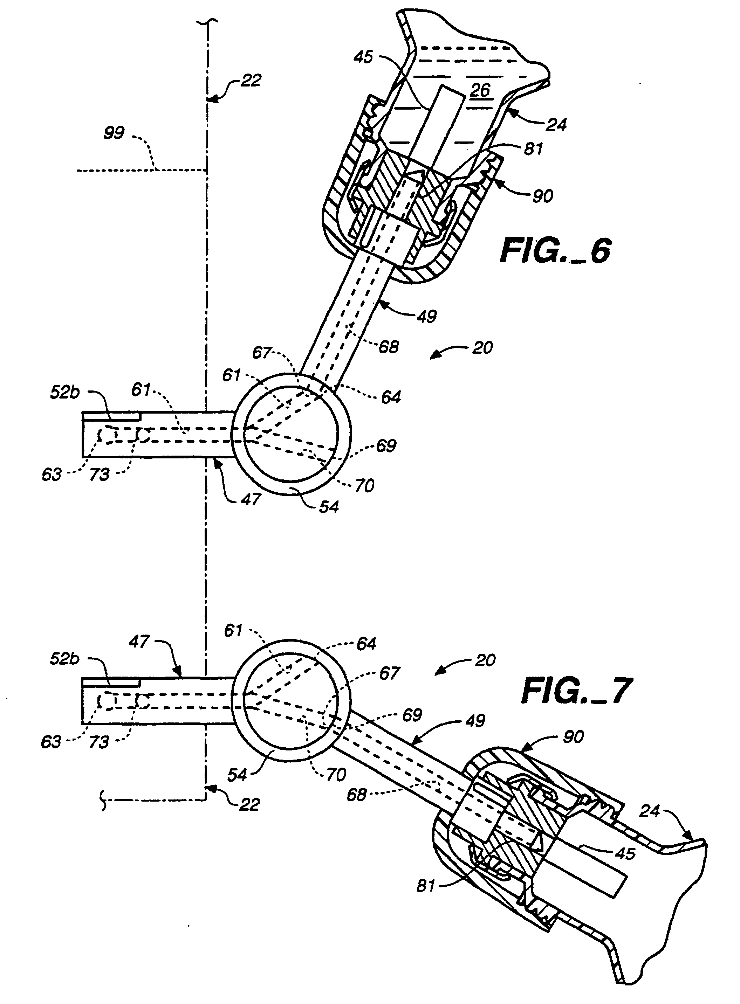

A connector of the present invention is designated generally by the reference number 20 in FIG. 1 and is adapted to be mounted on one end to an anesthetic vaporizer as schematically illustrated in FIGS. 4-8 by reference numeral 22. The other end of the connector 20 is connected to a container 24 which holds liquid anesthetic 26 (FIGS. 4 and 6). The connector 20 permits draining of the inverted container 24 (FIG. 6) into the vaporizer 22 to fill the internal reservoir of the vaporizer 22.

The container 24 defines an opening or discharge port 27 (FIG. 4). In a preferred container design, the port 27 is sealed with a closure 30. The closure 30 may be molded from a resilient material (such as polyethylene) and frictionally engaged with the container 24 at the opening 27. If desired, an adhesive or sealant compound could be applied to the interior of the container 24 in the opening 27 just prior to mounting the closure 30 in the container 24.

As shown in FIG. 4, the closure 30 includes a cylindrical inner portion 32 and a larger diameter, outer flange 34. The flange 34 is adapted to seat against the end of the container 24. Preferably, a sealing sleeve, band, or ferrule 38 is applied around the end of the container 24 and the closure flange 34 to insure an effective seal. The sleeve or band 38 may be a shrink fit material that has been shrunk into tight engagement with the exterior of the container 24 and closure flange 34.

The closure 30 also includes a collar 36 projecting outwardly from the flange 34 to define a receiving socket. An inside portion of the collar 36 defines a predetermined key or shaped structure, such as a rib (not visible) for engaging a groove in the connector 20 as described in detail hereinafter.

The closure 30 provides access to the interior of the container 24 through two, parallel ports -- one port 42 being visible in FIG. 4 in front of the other port 44. Port 42 functions as a discharge passage to drain the liquid anesthetic from the container 24, and port 44 functions as a vent passage for admitting gas (e.g., air) into the container 24 as the liquid contents discharge from the container through the port 42. To aid in the venting process, the closure 30 defines an inwardly projecting tube 45 which defines a continuation of the port 44.

In the initially manufactured condition, the closure 30 does not permit flow through the ports 42 and 44. To this end, the port 42 is occluded by a recessed, pierceable diaphragm or membrane 46 (FIG. 4), and the port 44 is occluded by a similar recessed, pierceable diaphragm or membrane (not visible in the Figures, but located in the port 44 at the same depth as the membrane 46).

The connector 20 has a pair of pivotably mounted couplings, a first coupling 47 and a second coupling 49 (Fig. 1). The first coupling 47 has a first leg 51 (FIG. 1) with a distal end 52 for connecting the coupling 47 to the vaporizer 22. In the embodiment illustrated, the end 52 defines a notch 52b which functions as a predetermined shape or key for mating with a corresponding or complementary structure (not illustrated) on the vaporizer 22.

The connector 20 is initially mounted to the vaporizer 22 by pushing the connector coupling 47 endwise into the vaporizer inlet port. A vaporizer designed for one type of anesthetic would have a particular configuration or key at its inlet port for mating with only one specific coupling shape of the connector 20. In this way, only a connector intended for one type of anesthetic can be connected to a vaporizer designed for that anesthetic.

The first leg 51 of the first coupling 47 has a proximal end defining a cylindrical portion 53. The cylindrical portion 53 is received in an annular wall 54 at the distal end of the second coupling 49. These parts of the two couplings function as a combination valve and mounting structure as explained in detail hereinafter.

The second coupling 49 includes a leg 56 projecting from the annular wall 54 and terminates in an enlarged boss or plug 60. The plug 60 is adapted to be received in the socket defined by the container closure collar 36 as shown in FIG. 5. A peripheral portion of the plug 60 defines a recess 60a which has a shape that is complementary to, and that is adapted to mate with, a rib (not visible) on the inside of the container closure collar 36.

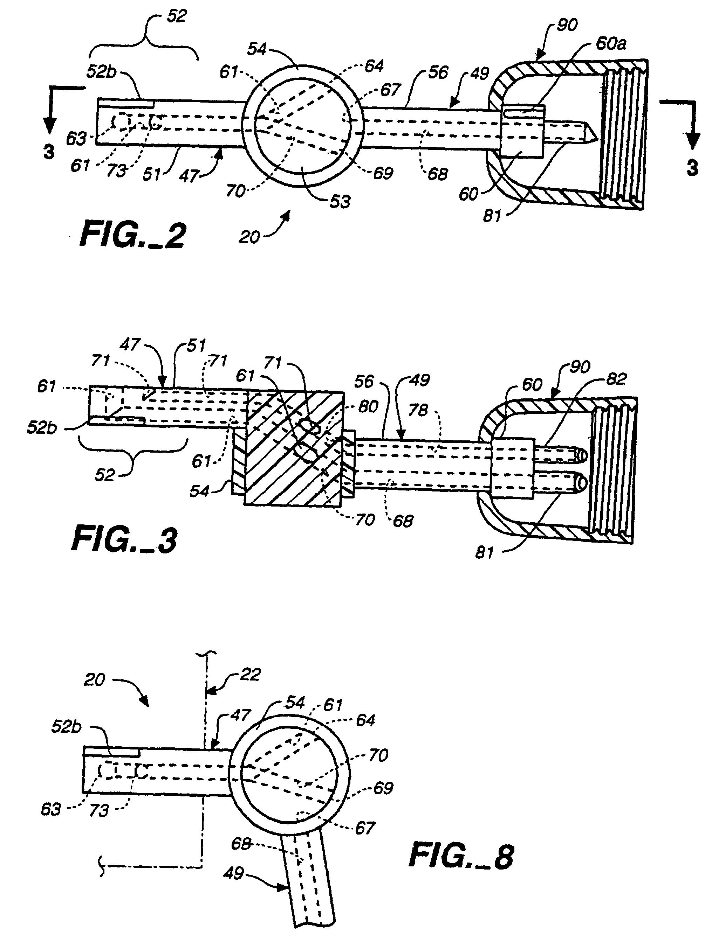

As shown in FIGS. 1 and 3, the first coupling 47 defines a first through passage 61. The first coupling 47 also defines a discharge port 63 at one end of the first passage 61 and defines an inlet port 64 at the other end of the first passage 61.

The second coupling 49 defines an influent port 66 and an outlet port 67 connected via a second passage 68. The first passage 61 and the second passage 68 are adapted to be selectively placed in communication by pivoting the second coupling 49 as explained hereinafter in detail.

The first coupling 47 also defines a drain port 69 and a third passage 70 which diverges from the first passage 61 and communicates with the port 69. Pivoting of the second coupling 49 to a particular position will establish communication between the third passage 70 and the second passage 68 as explained below in detail.

The first coupling 47 further defines a fourth through passage 71. A first gas vent port 73 is defined at one end of the fourth passage 71, and a second gas vent port 74 is defined at the other end of the fourth passage 71.

The second coupling 49 also defines a first gas vent port 76 and a second gas vent port 77 connected via a fifth passage 78. The fourth passage 71 and the fifth passage 78 are adapted to be placed in communication whenever the second coupling 49 is pivoted to establish communication between the first passage 61 and the second passage 68 as explained below in detail.

Finally, the first coupling 47 defines a drain vent port 79 and a sixth passage 80 which diverges from the fourth passage 71 and communicates with the port 79. When the second coupling 49 is pivoted to establish communication between the third passage 70 and the second passage 68, communication between the sixth passage 80 and the fifth passage 78 is established as explained in detail hereinafter.

As can be seen in FIGS. 1 and 3, the diverging pair of passages 71 and 80 and the diverging pair of passages 61 and 70 are angled within the cylindrical portion 53. This enables the second coupling outlet port 67 to be aligned with either the first coupling inlet port 64 or the first coupling drain port 69 -- depending on the rotational orientation of the second coupling 49. Similarly, the second coupling gas vent port 76 can be aligned with either the first coupling gas vent port 74 or the first coupling drain vent port 79 -- depending on the rotational orientation of the second coupling 49.

As illustrated in FIG. 1, the plug 60 includes a first projecting conduit 81 through which a portion of the passage 68 extends. The conduit 81 defines a hollow piercing tip at the port 66 for entering into the closure port 42 to pierce the membrane 46. The plug 60 also defines a second projecting conduit 82 through which a portion of the passage 78 extends. The conduit 82 defines a hollow piercing tip at the port 77 for entering into the closure port 44 to pierce the membrane therein (that membrane not being visible in the figures).

The plug 60 is adapted to engage the container 24 after the connector 20 has been properly connected with the vaporizer 22. First the connector second coupling 49 is pivoted to a downwardly angled, "closed" position shown in FIG. 4. In this position the second coupling gas vent port 76 and liquid outlet port 67 are occluded by the convex surface of the cylindrical portion 53 of the first coupling 47.

As illustrated in FIG. 4, the container 24 can then be located to align the socket of the closure collar 36 with the connector plug 60. The plug recess 60a is aligned with a mating closure rib (not visible) in the closure socket, and the ports 42 and 44 are aligned with the piercing conduits 81 and 82, respectively. Then, the container 24 can be pushed upwardly slightly so that the piercing conduits 81 and 82 begin to enter the ports 42 and 44, respectively.

The size and shape of each conduit 81 and 82 adjacent its tip is effective to establish a liquid-tight seal around the conduit at the port as the tip begins to engage the membrane (e.g., membrane 46 in FIG. 4). The proper and complete upward displacement of the container 24 relative to the connector 20 is effected by a system which includes a collar 90 on the connector 20. The collar 90 is disposed for rotation on the connector leg 56. The inner end of the collar 90 is adapted to engage the plug 60 (FIG. 4) which has a diameter larger than the diameter of the leg 56.

The interior of the collar 90 defines a screw -thread 92, and a mating screw thread 96 is provided on the container 24. Alternatively, the mating screw thread 96 could be provided on the exterior of the closure 30 outwardly of the container 24 (and the collar 90 would have to be configured as necessary to properly engage such a screw thread on the closure).

As the threaded, swivel collar 90 is engaged with the container thread 96 and tightened on the container 24, the closure ports 42 and 44 are pulled onto the plug conduits 81 and 82, respectively, and the membranes (e.g., membrane 46) are pierced (FIG. 5). The membranes are sufficiently recessed so that they are not ruptured until after a liquid-tight seal is effected between the plug conduits 81 and 82 and the mating portions of the ports 42 and 44 which are exterior of the membranes.

Typically, the closure material, at least in the region of the ports 42 and 44 exterior of the membranes, is somewhat resilient. Further, the diameters of the ports 42 and 44, exterior of the membranes, are less than the maximum exterior diameters of the conduits 81 and 82, respectively. This ensures the formation of tight seals just prior to, as well as after, the piercing of the membranes.

Preferably, the membranes are formed as unitary portions of the closure. It is presently contemplated that the preferred closure will be molded as the unitary structure from a suitable thermoplastic material (e.g., polyethylene). The pierceable membranes are preferably molded as part of the closure in the form of generally round, frangible disks or diaphragms.

After the container 24 has been properly attached to the connector 20, the connector second coupling 49 is pivoted upwardly from the closed position illustrated in FIG. 5 to a "fill" position illustrated in FIG. 6. In the fill position, the second coupling outlet port 67 is aligned with the first coupling inlet port 64. The anesthetic liquid can thus flow sequentially through the passage 68, passage 61, and out of the discharge port 63 into the vaporizer 22.

Further, when the connector 20 is in the fill position illustrated in FIG. 6 with the container 24 attached, the vent conduit 45 within the container 24 communicates with the first coupling gas vent port 73 of the first coupling 47 mounted in the vaporizer (FIG. 6). As can be seen in FIG. 1, gas can flow sequentially through the vent port 73 and fourth passage 71 to the vent passage 78 in the second coupling 49 which communicates through the inserted conduit 82 with the container vent tube 45 as shown in FIG. 5. Within the vaporizer 22 the gas vent port 73 communicates with air at atmospheric pressure to accommodate venting of air into the container 24 as the container contents 26 flow into the reservoir of the vaporizer 22.

As illustrated in FIG. 6, the reservoir of the vaporizer 22 has a nominal maximum fill elevation or level 99. Preferably, the second coupling 49 has a length sufficient to maintain the lowest part of the container 24 above the fill level elevation 99 of the vaporizer so that the container 24 can be completely emptied.

Vaporizers typically have a drain valve (not illustrated) which can be operated to open or close the inlet to the vaporizer reservoir. In the open position, it permits the vaporizer to be filled with the anesthetic liquid from the container. After the anesthetic liquid 26 has been drained from the container 24, the vaporizer valve can be closed to isolate the reservoir of anesthetic liquid in the vaporizer and prevent the liquid from draining back out through the first coupling discharge port 63. Then, the second coupling 49 can be pivoted downwardly from the "fill" position shown in FIG. 6 to a "drain" position shown in FIG. 7.

In the "drain" position, the second coupling passage 68 is in communication with the downwardly slanting passage 70 in the first coupling 47 (FIG. 7). Also, the vent passage 78 (FIGS. 1 and 2) in the second coupling 49 is in communication with the first coupling downwardly slanting passage 80 (in FIGS. 1 and 2). In the "drain" position, any residual anesthetic liquid in the connector 20 and in the upper region of the container 24 can drain to the bottom of the container 24.

In some situations when not all of the liquid anesthetic in the vaporizer has been used, it may be desirable to drain the remaining liquid anesthetic from the vaporizer reservoir. This is typically done by opening the valve on the vaporizer reservoir. In such a case, it would be especially useful to drain the vaporizer into the empty container 24 through the connector passages 61, 70, and 68. This can be accomplished by opening the vaporizer valve when the second coupling 49 is in the drain position.

Next, after any anesthetic liquid has drained back into the container 24, the second coupling 49 can be rotated to the "closed" position illustrated in FIGS. 5 and 8. In the closed position, the second coupling passages 68 and 78 are occluded by the first coupling cylindrical portion 53 so no drops of liquid can leak from the first coupling 47. The container 24 can then be unscrewed from the second coupling 49 and removed from the system. The connector 20 is then ready to receive a new container 24. The empty container 24 can be removed for disposal. An auxiliary cover (not illustrated) could be installed over the end of the empty container if desired.

Although not illustrated, travel stops and/or detents can be provided in the connector 20 so that the second coupling 49 can be self-maintained in each of the three positions (the "closed" position illustrated in FIGS. 1, 5, and 8; the "fill" position illustrated in FIG. 6; and the "drain" position illustrated in FIG. 7). Further, conventional or special sealing systems, such as "O"-ring seals, lands around the ports, etc., may be employed to eliminate, control, or minimize leakage.

It will also be appreciated that the connector 20 need not be removable from the vaporizer 22. The connector 20 may be permanently attached to, or formed as an integral or unitary part of, the vaporizer 22. In some applications, such a permanent attachment is preferred.

Although the connector 20 is illustrated as having a first coupling 47 that is keyed to mate with a complementary system on the vaporizer 22, it will be appreciated that such a keying system need not be provided for those systems where the keying advantages are not desired.

Further, the type of system for connecting the connector second coupling 49 to the container 24 illustrated in the figures need not be employed exclusively. Any other suitable connection system may be used, and the details of the system for connecting the second coupling 49 to a container 24 form no part of the present invention.

It will also be appreciated that the annular wall 54 of the second coupling 49 could be interchanged with the cylindrical portion 53 of the first coupling 47. That is, the wall 54 could be a unitary part of the first coupling 47, and the cylindrical portion 53 could be a unitary part of the second coupling 49. In such a case, the orientation of the pairs of diverging passages 61/70 and 71/80 would diverge from the second coupling passages 68 and 78, respectively.

It will be readily apparent from the foregoing detailed description of the invention and from the illustrations thereof that numerous variations and modifications may be effected without departing from the scope of the claims.

Where technical features mentioned in any claim are followed by reference signs, those reference signs have been included just for the sole purpose of increasing intelligibility of the claims and accordingly, such reference signs do not have any limiting effect on the scope of each element identified by way of example by such reference signs.

## Claims

1. A connector (20) suitable for mounting on an anesthetic vaporizer (22) for connecting the vaporizer (22) with a discharge port (27) of a container (24) of liquid anesthetic (26), the connector (20) comprising:
a first coupling attachable (47) to the vaporizer (22) and defining a first through passage (61) communicating at one end with the vaporizer (22);
a second coupling (49) attachable to the container (24) and defining a second through passage (68) communicating at one end with the container discharge port (27);
a third passage (70) defined by one (47) of the couplings (47, 49) and permanently communicating with one (61) of the first and second passages (61, 68); and
a combination valve and mounting structure cooperatively defined by the couplings (47, 49) to accommodate pivoting of the second coupling (49) to a closed position occluding the first and second passages (61, 68), to a raised fill position establishing communication between the first and second passages (61, 68), and to a lowered drain position in which the first and second passages (61, 68) communicate through the third passage (70),
**characterized in that** the raised fill position establishes direct communication between the first and second passages (61, 68); and by
an annular wall (54) provided at an end of the second coupling (49) which is opposite to the end communicating with the container discharge port (27) and by a cylindrical portion (53) provided at an end of the first coupling (47) which is opposite to the end communicating with the vaporizer (22), the cylindrical portion (53) being received in the annular wall (54), the annular wall (54) and the cylindrical portion (53) at the respective ends of the first and second couplings (47, 49) forming the valve structure.

2. The connector (20) of claim 1 wherein the first coupling (47) defines first and second gas vent ports (73, 74) connected via a fourth passage (71);
the second coupling (49) defines first and second gas vent ports (76, 77) connected via a fifth passage (78); and
one (47) of the couplings (47, 49) defines a drain vent port (79) and a sixth passage (80) diverging from one (71) of the fourth and fifth passages (71, 78) to communicate with the drain vent port (79) whereby
when the couplings (47, 49) are in the fill position, the fourth and fifth passages (71, 78) are in communication,
when the couplings (47, 49) are in the drain position, the sixth passage (80) communicates between the fourth and fifth passages (71, 78), and
when the couplings (47, 49) are in the closed position, flow between the fourth and fifth passages (71, 78) is occluded.

3. The connector (20) of claims 1 or 2 wherein the first coupling (47) is fixedly attached to the vaporizer (22).

4. The connector (20) of one of claims 1-3 wherein
the first coupling (47) defines a discharge port (63) and an inlet port (64) connected via the first passage (61);
the second coupling (49) defines an influent port (66) and an outlet port (67) connected via the second passage (68); and
one (47) of the couplings (47, 49) defines a drain port (69), the third passage (70) diverging from one (61) of the first and second passages (61, 68) to the drain port (69),
the first and second passages (61, 68) communicating in the fill position through the inlet port (64) of the first coupling (47) and the outlet port (67) of the second coupling (49).

5. The connector (20) of claim 4 wherein the first coupling (47) includes a first leg (51) having a distal end (52) for insertion into the vaporizer (22) and having the cylindrical portion (53) at a proximal end of the first leg (51); and
the distal end (52) of the first leg (51) defines the discharge port (63) and the cylindrical portion (53) defines the inlet port (64), the drain port (69), and the third passage (70) as a branch off of the first passage (61).

6. The connector (20) of claims 4 or 5 wherein the second coupling (49) includes a second leg (56) having a proximal end (60) for attaching to the container (24) and having the annular wall (54) at a distal end for rotatably receiving the cylindrical portion (53);
the second leg proximal end (60) defines the influent port (66); and
the annular wall (54) defines the outlet port (67).

7. The connector (20) of claim 6 wherein the cylindrical portion (53) projects beyond an end of the annular wall (54).

8. The connector (20) of claim 4 wherein the first coupling (47) and the second coupling (49) each include a key structure for permitting attachment only to a vaporizer (22) and container (24), respectively, that each have a mating key structure.

9. The connector (20) of claim 4 wherein the second coupling (49) includes
at least one projecting conduit (81) communicating with the second passage (68) and defining a piercing tip for entering a port (42) of the container (24) to pierce a membrane (46) therein, the size and shape of the conduit (81) adjacent the tip being effective to establish a liquid-tight seal around the conduit (81) at the container port (42) as the tip pierces the membrane (46); and
a collar (90) mounted for rotation on the second coupling (49), the collar (90) engaging the second coupling (49) and defining a screw thread (92) for engaging a thread (96) on the container (24) to pull the container port (42) onto the conduit (81) as increasing threaded engagement is established whereby the piercing of the membrane (46) is effected.

10. The connector (20) of claim 4 for use with the container (24) in which
the container (24) has a closure (30) which includes a receiving socket (36) communicating with the port (27);
the second coupling (49) has a plug that includes a boss adapted for insertion into the socket (36); and
the second coupling (49) has a conduit (81) which communicates with the second passage (68) and projects from the boss for entering into the port (27).

11. The connector (20) of claim 4 wherein the first coupling (47) has a generally rigid construction.

12. The connector of claim 4 wherein the connector (20) is generally Z-shaped.

## Patentansprüche

1. Ein Verbindungsteil (20), das für die Montage an einem Narkosemittelzerstäuber (22) geeignet ist, um den Zerstäuber (22) mit einer Abflussöffnung (27) eines Behälters (24) von flüssigem Narkosemittel (26) zu verbinden, wobei das Verbindungsteil (20) folgendes umfasst:
eine erste Kopplung (47), die am Zerstäuber (22) anbringbar ist, und die einen ersten durchgehenden Durchgang (61) bestimmt, der an einem Ende mit dem Zerstäuber (22) in Verbindung steht;
eine zweite Kopplung (49), die am Behälter (24) anbringbar ist, und die einen zweiten durchgehenden Durchgang (68) bestimmt, der an einem Ende mit der Abflussöffnung (27) des Behälters in Verbindung steht;
einen dritten Durchgang (70), der durch eine (47) der Kopplungen (47, 49) bestimmt wird, und der permanent mit einem (61) des ersten und zweiten Durchgangs (61, 68) in Verbindung steht; und
ein Kombinationsventil und einen Montageaufbau, die zusammenwirkend von den Kopplungen (47, 49) bestimmt werden, um die Drehung der zweiten Kopplung (49) in eine geschlossene Stellung, die den ersten und zweiten Durchgang (61, 68) verschließt, in eine erhöhte Füllstellung, die die Verbindung zwischen dem ersten und zweiten Durchgang (61, 68) herstellt, und in eine gesenkte Abflussstellung, in der der erste und zweite Durchgang (61, 68) über den dritten Durchgang (70) in Verbindung stehen, zu ermöglichen,
**dadurch gekennzeichnet, dass** die erhöhte Füllstellung die direkte Verbindung zwischen dem ersten und zweiten Durchgang (61, 68) herstellt; und durch
eine ringförmige Wand (54), die an einem Ende der zweiten Kopplung (49) bereitgestellt ist, das dem Ende entgegengesetzt ist, das mit der Abflussöffnung (27) des Behälters in Verbindung steht, und durch einen zylindrischen Abschnitt (53), der an einem Ende der ersten Kopplung (47) bereitgestellt ist, das dem Ende entgegengesetzt ist, das mit dem Zerstäuber (22) in Verbindung steht, wobei der zylindrische Abschnitt (53) in der ringförmigen Wand (54) aufgenommen wird, wobei die ringförmige Wand (54) und der zylindrische Abschnitt (53) an den jeweiligen Enden der ersten und zweiten Kopplung (47, 49), die den Ventilaufbau bilden, befindlich sind.

2. Das Verbindungsteil (20) gemäß Anspruch 1, worin die erste Kopplung (47) erste und zweite Gaslüftungsöffnungen (73, 74) bestimmt, die über einen vierten Durchgang (71) verbunden sind;
wobei die zweite Kopplung (49) erste und zweite Gaslüftungsöffnungen (76, 77) bestimmt, die über einen fünften Durchgang (78) verbunden sind; und
wobei eine (47) der Kopplungen (47, 49) eine Abflusslüftungsöffnung (79) und einen sechsten Durchgang (80) bestimmt, die von einem (71) des vierten und fünften Durchgangs (71, 78) abgezweigt ist, um mit der Abflusslüftungsöffnung (79) in Verbindung stehen, wodurch
wenn die Kopplungen (47, 49) in der Füllstellung befindlich sind, der vierte und fünfte Durchgang (71, 78) in Verbindung stehen,
wenn die Kopplungen (47, 49) in der Abflussstellung befindlich sind, der sechste Durchgang (80) zwischen dem vierten und fünften Durchgang (71, 78) in Verbindung steht, und
wenn die Kopplungen (47, 49) in der geschlossenen Stellung befindlich sind, die Strömung zwischen dem vierten und fünften Durchgang (71, 78) verschlossen ist.

3. Das Verbindungsteil (20) gemäß Anspruch 1 oder 2, worin die erste Kopplung (47) am Zerstäuber (22) fest angebracht ist.

4. Das Verbindungsteil (20) gemäß einem oder mehreren der Ansprüche 1-3, worin
die erste Kopplung (47) eine Abflussöffnung (63) und eine Einlassöffnung (64) bestimmt, die über den ersten Durchgang (61) verbunden sind;
wobei die zweite Kopplung (49) eine Einflussöffnung (66) und eine Auslassöffnung (67) bestimmt, die über den zweiten Durchgang (68) verbunden sind; und
wobei eine (47) der Kopplungen (47, 49) eine Abflussöffnung (69) bestimmt, wobei der dritte Durchgang (70) von einem (61) des ersten und zweiten Durchgangs (61, 68) zur Abflussöffnung (69) abgezweigt ist,
wobei der erste und zweite Durchgang (61, 68) in der Füllstellung durch die Einlassöffnung (64), der ersten Kopplung (47) und die Auslassöffnung (67) der zweiten Kopplung (49) in Verbindung stehen.

5. Das Verbindungsteil (20) gemäß Anspruch 4, worin die erste Kopplung (47) einen ersten Schenkel (51) einschließt, der ein distales Ende (52) für die Einführung in den Zerstäuber (22) hat, und wobei der zylindrische Abschnitt (53) an einem proximalen Ende des ersten Schenkels (51) befindlich ist; und
wobei das distale Ende (52) des ersten Schenkel (51) eine Abflussöffnung (63) bestimmt und der zylindrische Abschnitt (53) die Einlassöffnung (64) die Abflussöffnung (69) und den dritten Durchgang (70) als eine Abzweigung des ersten Durchlasses (61) bestimmt.

6. Das Verbindungsteil (20) gemäß Anspruch 4 oder 5, worin die zweite Kopplung (49) einen zweiten Schenkel (56) einschließt, der ein proximales Ende (60) für die Anbringung am Behälter (24) hat, und wobei die ringförmige Wand (54) an einem distalen Ende befindlich ist, um den zylindrischen Abschnitt (53) drehbar aufzunehmen;
wobei das proximale Ende (60) des zweiten Schenkels die Einflussöffnung (66) bestimmt; und
wobei die ringförmige Wand (54) die Auslassöffnung (67) bestimmt.

7. Das Verbindungsteil (20) gemäß Anspruch 6, worin der zylindrische Abschnitt (53) über ein Ende der ringförmigen Wand (54) hinausragt.

8. Das Verbindungsteil (20) gemäß Anspruch 4, worin sowohl die erste Kopplung (47) als auch die zweite Kopplung (49) einen Schlüsselaufbau einschließt, um nur die Anbringung an einem jeweiligen Zerstäuber und Behälter zu gestatten, die beide einen passenden Schlüsselaufbau haben.

9. Das Verbindungsteil (20), gemäß Anspruch 4, worin die zweite Kopplung (49) folgendes einschließt:
mindestens eine hinausragende Leitung (81), die mit dem zweiten Durchgang (68) in Verbindung steht, und eine Durchstechspitze zum Eintritt in eine Öffnung (42) des Behälters (24) bestimmt, um eine Membran (46) darin durchzustechen, wobei die Größe und Form der Leitung (81) in der Nähe der Spitze wirksam ist, um eine flüssigkeitsdichte Dichtung um die Leitung (81) herum an der Behälteröffnung (42) herzustellen, wenn die Spitze die Membran (46) durchsticht; und
eine Manschette (90), die für Drehung auf der zweiten Kopplung (49) angebracht ist, wobei die Manschette (90) in die zweite Kopplung (49) eingreift und ein Schraubengewinde (92) bestimmt, um in ein Gewinde (96) auf dem Behälter (24) einzugreifen, um die Behälteröffnung (42) in die Leitung (81) zu ziehen, wenn ein erhöhter Schraubeingriff hergestellt wird, wodurch das Durchstechen der Membran (46) durchgeführt wird.

10. Das Verbindungsteil (20) gemäß Anspruch 4 zur Verwendung mit einem Behälter (24), in dem
der Behälter (24) einen Verschluss (30) hat, der eine Muffe (36) hat, die mit der Öffnung (27) in Verbindung steht;
die zweite Kopplung (49) einen Stecker hat, der einen Knopf einschließt, der für die Einführung in die Muffe (36) ausgebildet ist; und
die zweite Kopplung (49) eine Leitung (81) hat, die mit dem zweiten Durchgang (68) in Verbindung steht und vom Knopf hinausragt, um in die Öffnung (27) einzutreten.

11. Das Verbindungsteil (20) gemäß Anspruch 4, worin die erste Kopplung (47) einen allgemein starren Aufbau hat.

12. Das Verbindungsteil gemäß Anspruch 4, worin das Verbindungsteil (20) allgemein Z-förmig ist.

## Revendications

1. Connecteur (20) adapté pour être monté sur un évaporateur d'anesthésique (22) pour raccorder l'évaporateur (22) à un orifice de décharge (27) d'un récipient (24) d'anesthésique liquide (26), le eonnecteur (20) comprenant :
un premier raccord (47) pouvant être fixé à l'évaporateur (22) et définissant un premier passage traversant (61) communiquant à une extrémité avec l'évaporateur (22) ;
un deuxième raccord (49) pouvant être fixé au récipient (24) et définissant un deuxième passage traversant (68) communiquant à une extrémité avec l'orifice de décharge (27) du récipient ;
un troisième passage (70) défini par l'un (47) des raccords (47, 49) et communiquant en permanence avec l'un (61) des premier et deuxième passages (61, 68) ; et
une structure de valve et de montage combinée définie en coopération par les raccords (47, 49) pour permettre le pivotement du deuxième raccord (49) vers une position fermée qui ferme les premier et deuxième passages (61, 68), vers une position élevée de remplissage établissant une communication entre les premier et deuxième passages (61, 68), et vers une position abaissée de vidange dans laquelle les premier et deuxième passages (61, 68) communiquent par l'intermédiaire du troisième passage (70),
caraetérisé en ce que la position élevée de remplissage établit une communication directe entre les premier et deuxième passages (61, 68) ; et par
une paroi annulaire (54) prévue à une extrémité du deuxième raccord (49) qui est opposée à l'extrémité communiquant avec l'orifice de décharge (27) du récipient et par une partie cylindrique (53) prévue à une extrémité du premier raccord (47) qui est opposée à l'extrémité communiquant avec l'évaporateur (22), la partie cylindrique (53) étant reçue dans la paroi annulaire (54), la paroi annulaire (54) et la partie cylindrique (53) aux extrémités respectives des premier et deuxième raccords (47, 49) formant la structure de valve.

2. Connecteur (20) selon la revendication 1, dans lequel le premier raccord (47) définit des premier et deuxième évents de gaz (73, 74) reliés par l'intexmédiaire d'un quatrième passage (71) ;
le deuxième raccord (49) définit des premier et deuxième évents de gaz (76, 77) reliés par l'intermédiaire d'un cinquième passage (78) ; et
l'un (47) des raccords (47, 49) définit un évent de vidange (79) et un sixième passage (80) divergeant de l'un (71) des quatrième et cinguième passages (71, 79) afin de communiquer avec l'évent de vidange (79) de telle manière que
lorsque les raccords (47, 49) sont dans la position de remplissage, les quatrième et cinquième passages (71, 78) soient en communication,
lorsque les raccords (47, 49) sont dans la position de vidange, le sixième passage (80) établisse une communication entre les quatrième et cinquième passages (71, 78), et
lorsque les raccords (47, 49) sont dans la position fermée, l'écoulement entre les quatrième et cinquième passages (71, 78) soit empêché.

3. Connecteur (20) selon les revendications 1 ou 2, dans lequel le premier raccord (47) est attaché fixement à l'évaporateur (22).

4. Connecteur (20) selon l'une des revendications 1 à 3, dans lequel
le premier raccord (47) définit un orifice de décharge (63) et un orifice d'entrée (64) reliés par l'intermédiaire du premier passage (61) ;
le deuxième raccord (49) définit un orifice d'amenée (66) et un orifice de sortie (67) reliés par l'intermédiaire du deuxième passage (68) ; et
l'un (47) des raccords (47, 49) définit un orifice de vidange (69), le troisième passage (70) divergeant de l'un (61) des premier et deuxième passages (61, 68) vers l'orifice de vidange (69),
les premier et deuxième passages (61, 68) communiquant dans la position de remplissage par l'intermédiaire de l'orifice d'entrée (64) du premier raccord (47) et de l'orifice de sortie (67) du deuxième raccord (49).

5. Connecteur (20) selon la revendication 4, dans lequel le premier raccord (47) comprend une première branche (52) comportant une extrémité distale (52) destinée à être insérée dans l'évaporateur (22) et comportant la partie cylindrique (53) à une extrémité proximale de la première branche (51) ; et
l'extrémité distale (52) de la première branche (51) définit l'orifice de décharge (63) et la partie cylindrique (53) définit l'orifice d'entrée (64), l'orifice de vidange (69) et le troisième passage (70) en tant que bifurcation du premier passage (51).

6. Connecteur (20) selon la revendication 4 ou 5, dans lequel le deuxième raccord (49) comprend une deuxième branche (56) comportant une extrémité proximale (60) destinée à être fixée au récipient (24) et comportant la paroi annulaire (54) à une extrémité distale pour recevoir de manière rotative la partie cylindrique (53) ;
l'extrémité proximale (60) de la deuxième branche définit l'orifice d'amenée (66) ; et
la paroi annulaire (54) définit l'orifice de sortie (67).

7. Connecteur (20) selon la revendication 6, dans lequel la partie cylindrique (53) s'étend au-delà d'une extrémité de la paroi annulaire (54).

8. Connecteur (20) selon la revendication 4, dans lequel le premier raccord (47) et le deuxième raccord (49) comprennent chacun une structure de verrouillage pour permettre la fixation uniquement à un évaporateur (22) et à un récipient (24), respectivement, qui comportent chacun une structure de verrouillage correspondante.

9. Connecteur (20) selon la revendication 4, dans lequel le deuxième raccord (49) comprend :
au moins un conduit saillant (81) communiquant avec le deuxième passage (68) et définissant une pointe de perforation destinée à pênétrer dans un orifice (42) du récipient (24) afin de perforer une membrane (46) à l'intérieur de celui-ci, la dimension et la forme du conduit (81) à proximité de la pointe étant efficaces pour établir un joint étanche aux liquides autour du conduit (81) au niveau de l'orifice (42) du récipient alors que la pointe perfore la membrane (46) ; et
un collier (90) monté pour rotation sur le deuxième raccord (49), le collier (90) étant en prise avec le deuxième raccord (49) et définissant un filet (92) destiné à étre mis en prise avec un filet (96) sur le récipient (24) afin de tirer l'orifice (42) du récipient sur le conduit (81) alors qu'une mise en prise par filetage croissante est établie de telle manière que la perforation de la membrane (46) soit effectuée.

10. Connecteur (20) selon la revendication 4 destiné à être utilisé avec le récipient (24), dans lequel
le récipient (24) comporte une fermeture (30) qui comprend un élément femelle de réception (36) communiquant avec l'orifice (27) ;
le deuxième raccord (49) comporte un élément mile qui comprend un bossage adapté pour être inséré dans l'élément femelle (36) ; et
le deuxième raccord (49) comporte un conduit (81) qui communique avec le deuxième passage (68) et s'étend depuis le bossage pour pénétrer dans l'orifice (27).

11. Connecteur (20) selon la revendication 4, dans lequel le premier raccord (47) présente une construction généralement rigide.

12. Connecteur selon la revendication 4, dans lequel le connecteur (20) présente généralement la forme d'un Z.
